# EUROPEAN PATENT APPLICATION

(11) **EP 2 272 383 A1**
(43) Date of publication of application: **12.01.2011**
(21) Application number: 09163349.5
(22) Date of filing: 22.06.2009
(51) Int. Cl.: A23L 1/30, A61K 8/97, A61K 36/02, A61K 8/36

(54) **Composition Comprising Omega-7 and/or Omega-4 Fatty Acids**

(71) Applicant: SBAE Industries NV, 9950 Waarschoot (BE)
(72) Inventor: Vanhoutte, Koenraad, 9040, St-Amandsberg (BE); Vanhoutte, Jan, 8750, Zwevezele (BE)
(74) Representative: BiiP cvba

(57) **Abstract**

The present invention is directed to an omega fatty acid composition comprising one or more of a fatty acid selected from the group of omega-4 fatty acids and omega-7 fatty acids, **characterized in that** omega-4 and omega-7 fatty acids are present in at least 10% weight percentage of the total fatty acid fraction (TFAF) of the composition.

The present invention is also directed to a process for obtaining the composition according to the invention, **characterized in that** the omega fatty acids are cultivated and stored in an algae biomass, either or not followed by extraction.

The present invention is also directed to the use of a composition according to the invention in at least 10%, preferably 30% and more preferably 50% weight percentage of the total weight.

## Description

### FIELD OF THE INVENTION

The present invention concerns a composition containing polyunsaturated fatty acids. More particular, it relates to a novel composition containing, as effective ingredients, omega-4 and omega-7 fatty acids, and the nutritionally, therapeutically and/or cosmetically acceptable derivates thereof.

### BACKGROUND OF THE INVENTION

The long-chain poly-unsaturated fatty acids are well known for their nutritional importance. As these acids confer flexibility, fluidity and selective permeability properties to cellular membranes the acids have been shown to be vital for brain development, beneficial for the cardiovascular, nervous, gastro-intestinal, musculoskeletal, metabolic, respiratory and/or the uro-genital system and for other important nutraceutical and pharmaceutical targets in human and animal health. Unsaturated fatty acid compositions can further be used for treating allergy disorders, inflammation or immunologic disorders and/or tumors. These acids are also used in dermatologic and/or cosmetic products. Functional food and feed is another embodiment of the present invention.

Membrane-associated processes are significant components of metabolism, and the acyl composition of membrane bilayers is associated with metabolic activity in a predictable manner. The relative balance between monounsaturated and long-chain polyunsaturated acyl chains in membrane bilayers is a fundamental determinant of metabolic rate of a species. The maximum possible length of life of an organism is a species characteristic. There is a link between body size, metabolic rate and lifespan of a species. Membrane bilayer composition may have a role in determining this maximum lifespan (MLSP). Whilst the physical properties imparted to membranes by their acyl chains influence metabolic rate, it is their chemical properties that are responsible for their role in aging. Via their role in lipid peroxidation, membrane acyl composition is related to maximum lifespan in mammals and birds. The acyl composition of the diet further significantly alters the metabolic rate. The oxidative stress theory hypothesizes that oxygen-derived free radicals are responsible for the age-related damage at the cellular and tissue levels. In a normal situation, a balanced-equilibrium exists among oxidants, antioxidants and biomolecules. Excess generation of free radicals may overwhelm natural cellular antioxidant defences leading to oxidation and further contributing to cellular functional impairment. The identification of free radical reactions as promoters of the aging process implies that interventions aimed at limiting or inhibiting them should be able to reduce the rate of formation of aging changes with a consequent reduction of the aging rate and disease pathogenesis.

The membrane acyl composition is related to maximum lifespan via their role in lipid peroxidation. To protect the cell against the result of accumulated damage, such as from reactive oxygen species (ROS) that are an inevitable byproduct of mitochondrial oxygen consumption, the acyl composition needs saturated and mono-unsaturated acyl chains lacking the carbon atoms between the -C=C- units found in polyunsaturated acyl chains and which are most susceptible to oxidative attack. Monounsaturated fatty acids, such as C16:1 can function as an anti-oxidant and scavenge oxidative damaging products such as reactive oxygen species (ROS).

Poly-unsaturated fatty acids are most susceptible to lipid peroxidation, in particular the carbon between two double bonds is susceptible to oxidation. Therefore monounsaturated fatty acids (MUFA's) are much less prone to oxidative damage than are poly-unsaturated fatty acids (PUFA's). It has surprisingly been found that changes in membrane acyl composition of both mitochondria and microsomes result in a decreased susceptibility of these membrane bilayers to lipid peroxidation.

Mono-unsaturated fatty acids, such as omega-7 and omega-4 fatty acids can function as an anti-oxidant and scavenge reactive oxygen species (ROS). By increasing the level of mono-unsaturated fatty acids in the cell membrane, cell peroxidation is decreased and thus cell protection and integrity is increased. As cells build up the several tissues of organisms, an increased amount of mono-unsaturated fatty acids confers whole organism protection, integrity and cell and tissue quality improvement. As the relative balance between mono-unsaturated and long-chain poly-unsaturated acyl chains in membrane bilayers is a fundamental determinant of metabolic rate of a species, the membrane acyl composition is very important for cell integrity, cell protection and cell restoration. It is thus of utmost importance to keep the right balance between mono-unsaturated fatty acids and poly-unsaturated fatty acids (resp. MUFA's and PUFA's), as well as the right mixture of omega's 3, 4 and 7 of various lengths.

Omega-7 fatty acids are mono-unsaturated fatty acids present in the phospholipid bilayer of the cell membrane. Hence, these acids are present in all tissues of the human body. Delicate body tissues, like cardiovascular tissue, skin and membranes that line the digestive and uro-genital tracts, have the greatest affinity for omega-7 fatty acids. Environmental stressors, improper foods, and normal aging can challenge those sensitive membranes, and omega-7 fatty acids are now being hailed as an agent to protect, replenish, moisturize, and restore.

Omega-4 fatty acids are also important in cell membrane composition. Their increased presence in membranes increases for instance fluidity and protection to salt stress and freezing. Moreover, increased unsaturation is beneficial in processes such as protein sorting mediated by lipid rafts in the membrane. This improves the cell functionality and increases the protection of the cell against cellular damages. Further, omega-4 fatty acids have anti-oxidative properties, anti-tumor promoting and anti-inflammatory protective activities after cell damage (against possible pathogens).

It has been reported that omega-7 fatty acids are precursors for the production of omega-4 fatty acids. Omega-4 16:2 or palmitolinoleic acid is synthesized from palmitoleic acid or omega-7 16:1. The pathways of both types of omega fatty acids are linked. Very likely they can in part fulfill similar functions. Rather stating the obvious but there is probably no such thing as an omega-4 or omega-7 or even omega-3 line, but rather a vast network of interactions that can switch from one end to the other albeit across intermediates. Clearly they are interrelated in a metabolically significant way. Omega-4 and omega-7 fatty acids are balanced and interchangeable within the cell membrane.

At present, natural sources of omega-7 fatty acids are mainly vegetal oils, such as Gevuina (Gevuina avellana), Macadamia (Macadamia integrifolia) and Sea Buckthorn (Hippophae rhamnoides) oils; although a variety of animal oils and marine oils also exist. Sources of omega-4 are fish oil, fungi, such as phytophora and mucorales, and soybean oil.

A limitation of using animal sources for omega-7 and/or omega-4 fatty acids is that the fatty acids are subject to seasonal fluctuations within species as well as differences between species. Literature indicates that this may vary more than tenfold. Another inconvenience is the complex regulations to be followed when dealing with material derived from animals. Further, animal sources may not be acceptable for people following certain food regimes (dietary or religious). Although using plant sources as a source for omega-7 and/or omega-4 fatty acids overcomes some disadvantages, typical bottle necks in production are the geographical limitations and the seasonal dependency for collecting the harvest. Seasonal dependency results in a discontinued production process and hence, causes economic losses. Also, growing terrestrial crops as a source is done according to conventional agriculture techniques which intensively use herbicides, pesticides and fertilizers. Conventional agriculture is furthermore very energy consuming and is bound to use of fresh water. There is a limited area of agricultural land available.

An alternative omega fatty acid source is microorganisms. Mass production of microorganisms, in particular high metabolic microorganisms such as algae, is possible in a continuous and sustainable way. The present invention serves as to provide a solution to one or more of the above mentioned drawbacks. Microalgae are an innovative natural and vegetal omega fatty acids source with a high added value. Algae are a renewable resource and are optimal in nature preservation and to sustain an environmental friendly process. No fresh water is needed to grow microorganisms, hence, sustainability in the waterbalance of process water in the production process is guaranteed. Further, only limited production area is needed and production can even be done using waste land. Microalgae are not only the main source of oxygen on Earth; they also absorb CO2 through photosynthesis. Microalgae cultures contribute to reduce atmospheric pollution, as well as nutrient overload in rivers and oceans. Microalgae offer an economic benefit as the microalgae production process is ten times faster than for conventional terrestrial plants. Microalgae production requires tenfold less fertilizers and is essentially feasible without the use of herbicides nor pesticides. The production process is further continuous as there are no seasonal growth limitations which result in a start-stop scenario i.e. seed to harvest cycle.

Hence, the present invention provides a composition comprising omega-7 and omega-4 fatty acids, **characterized in that** the omega 4/7 fatty acid composition is extracted from algae, particularly diatoms. Diatoms are unicellular plants broadly present in freshwater and marine ecosystems, where they play a primary role in sustaining the aquatic food chain.

It has been surprisingly found that, in accordance with the present invention, microorganisms, preferably algae and more preferably diatoms can be used as a source for extracting omega-7 and omega-4. Further, it has been found that omega-7 and omega-4 fatty acids can be used to decrease cell membrane peroxidation and acts as an active ingredient for the maintenance of cell integrity and cell protection. They are also essential in the regulation of the effects of the metabolic rate of the cells.

Accordingly, it is an object of the present invention to provide a composition comprising omega-7 and omega-4 fatty acids, either or not combined with other omega fatty acids, for cell protection by limiting cell peroxidation, tissue protection and cell and tissue quality improvement and restoration.

It is a further objective to provide a solution for the several daily stress factors the body suffers from, such as ageing accompanied with lipid peroxidation. The present invention further provides a topical and/or a 'per os' solution to provide the multicellular organisms with additional protecting mono-unsaturated fatty acids as an active ingredient in a nutrient, therapeutic and/or cosmetic product.

Another objective is to yield a higher bio-activity by combining the omega-7 and omega-4 fatty acids with other fatty acids, such as omega-3, omega-6, omega-9, omega-11 and/or omega-13 fatty acids. When used in conjunction with certain other bio-activity exhibiting compounds, a lower concentration of active component is needed by the synergistic effects of the different active compounds. The combined use of omega-4 and omega-7 fatty acids with poly-unsaturated fatty acids, such as omega-3, omega-6, omega-9, omega-11 and omega-13 fatty acids, results in higher or similar bioactivities, in part because of the physical-chemical properties of the omega fatty acids, and with reduced negative effect which results from the high oxidizability of poly-unsaturated fatty acids and which is now more protected by the 04 and 07's on a chemical level.

It is yet a further object of the present invention to provide a process for obtaining a composition comprising omega-7 and omega-4 fatty acids, whereby the microorganisms are cultivated and stored in an algae biomass, either or not followed by extraction.

### SUMMARY OF THE INVENTION

The present invention is directed to an omega fatty acid composition comprising one or more of a fatty acid selected from the group of omega-4 fatty acids and omega-7 fatty acids, **characterized in that** omega-4 and omega-7 fatty acids are present in at least 10% weight percentage of the total fatty acid fraction (TFAF) of the composition.

The present invention is also directed to a process for obtaining the composition according to the invention, **characterized in that** the omega fatty acids are cultivated and stored in an algae biomass, either or not followed by extraction.

The present invention is also directed to the use of a composition according to the invention in at least 10%, preferably 30% and more preferably 50% weight percentage of the total weight.

### DETAILED DESCRIPTION OF THE INVENTION

The oxidative stress theory hypothesizes that oxygen-derived free radicals are responsible for the age-related damage at the cellular and tissue levels. In a normal situation, a balanced-equilibrium exists among oxidants, antioxidants and biomolecules. Excess generation of free radicals may overwhelm natural cellular antioxidant defenses leading to oxidation and further contributing to cellular functional impairment. The identification of free radical reactions as promoters of the aging process implies that interventions aimed at limiting or inhibiting them should be able to reduce the rate of formation of aging changes with a consequent reduction of the aging rate and disease pathogenesis.

The ROS superoxide anion is involved in clinical conditions related to for instance the gastrointestinal system (e.g. hepatitis, liver injury), the eyes (e.g. cataractogenesis, retinal damage, AMD), the skin (e.g. dermatitis, age pigment), the heart (e.g. heart disease), the teeth (e.g. periodontis), the cardiovascular system (e.g. atherosclerosis, vasospasms), multiorgan failure (e.g. cancer), the cerebral system (e.g. trauma, stroke) and the respiratory system (e.g. asthma, hyperoxia). Antioxidant supplementation might improve major outcomes of interest in humans and animals (ie, physical performance, muscle strength, longevity).

Proteins, carbohydrates, nucleic acids and lipids are all targets of such oxidative damage. In lipids, it is the carbon atoms between the -C=C- units found in polyunsaturated acyl chains that are most susceptible to oxidative attack. Saturated and monounsaturated acyl chains lack such carbon atoms. The poly-unsaturated fatty acids, such as long-chain poly-unsaturates in membrane bilayers of mitochondria, however, are very susceptible to damage, both chemically and also because of their location close to the site of ROS production. Membrane lipid peroxidation is an autocatalytic chain reaction, and many of its products are themselves very potent damagers of proteins.

In an embodiment of the present invention, an omega fatty acid composition is provided comprising one or more of a fatty acid selected from the group of omega-4 fatty acids and omega-7 fatty acids, **characterized in that** omega-4 and omega-7 fatty acids are present in at least 10% weight percentage of the total fatty acid fraction of the composition. Uptake of this composition by the cell membranes increases the protection of the cell against lipid peroxidation and improves thus the cell integrity and cell quality.

In a further embodiment of the present invention, the composition is extracted from microorganisms. Mass production of microorganisms, in particular algae, and more preferably diatoms, is possible in a continuous and sustainable way.

As the composition is beneficial for all cells and all tissues in the human and/or animal body, there are many disorders that can be treated with the composition provided in the invention. The composition restores imbalances in the saturated /mono-unsaturated/poly-unsaturated lipid profile of the cell membrane and increases the level of mono-unsaturated lipids, whereby lipid peroxidation is controlled and decreased. The present invention is directed to a topical and/or oral product, with a special focus on nutrients, therapeutic and cosmetic products. More preferably, the invention focuses on a product for treating disorders of the cardiovascular, cerebral, nervous, gastro-intestinal, musculoskeletal, metabolic, respiratory and/or uro-genital system. The product may further treat allergy disorders, inflammation or immunologic disorders and/or tumors. Another embodiment of the invention provides a composition used in dermatologic and/or cosmetic products.

### DESCRIPTION OF THE PREFERRED EMBODIMENT

According to the present invention, an omega fatty acid composition is provided comprising one or more of a fatty acid selected from the group of omega-4 fatty acids and omega-7 fatty acids, **characterized in that** omega-4 and omega-7 fatty acids are present in at least 10% weight percentage of the total fatty acid fraction of the composition.

To perform its metabolic functions, the cell needs to be protected against endogenous and exogenous factors that can have deleterious effects. As a consequence, it is highly desirable to facilitate and increase the amount of cell protection in the human body.

Lipids play varied and critical roles in metabolism, with function dramatically modulated by the individual fatty acid moieties in complex lipid entities. In particular, the fatty acid composition of membrane lipids greatly influences membrane function. The cell membrane consists of three classes of amphipathic lipids: phospholipids, glycolipids, and steroids. The amount of each depends upon the type of cell, but in the majority of cases phospholipids are the most abundant. The fatty chains in phospholipids and glycolipids usually contain an even number of carbon atoms, typically between 16 and 20. The 16- and 18-carbon fatty acids are the most common. Fatty acids may be saturated or unsaturated, with the configuration of the double bonds nearly always cis. The backbone of most membrane lipids is the three-carbon glycerophosphate, and the membrane lipids consist of fatty acyl chains ester-linked to this three-carbon backbone. The three acyl chains normally synthesized are palmitate (16:0), palmitoleate (16:1, n-7) and vaccenate (18:1, n-7).

The acyl chains are saturated, mono-unsaturated or poly-unsaturated hydrocarbon chains. The length and the degree of unsaturation of fatty acid chains have a profound effect on membranes fluidity as unsaturated lipids create a kink, preventing the fatty acids from packing together tightly, thus decreasing the melting point (increasing the fluidity) of the membrane. They thus confer flexibility, fluidity and selective permeability properties to cellular membranes.

However, the poly-unsaturated fatty acids, such as long-chain poly-unsaturates in membrane bilayers of mitochondria are very susceptible to damage, both chemically and also because of their location close to the site of reactive oxygen species (ROS) production. ROS are an inevitable byproduct of mitochondrial oxygen consumption. Membrane lipid peroxidation is an autocatalytic chain reaction, and many of its products are themselves very potent damagers of proteins. As the carbon atoms between the -C=C- units found in poly-unsaturated acyl chains are most susceptible to oxidative attack, the acyl membrane composition needs saturated and mono-unsaturated acyl chains lacking such carbon atoms to protect the cell against the result of accumulated damage from ROS.

It has been proposed that both the amount of membranes and their acyl composition, notably the relative balance between mono-unsaturated and poly-unsaturated acyl chains, are a pacemaker for metabolic activity and basal metabolism. The evidence supporting the 'membrane pacemaker theory' is of two types: (1) the acyl composition of membrane bilayers varies in a manner similar to variations in metabolic rate and (2) variations in acyl composition of membrane bilayers influence membrane-associated processes.

To perform its metabolic functions, the cell needs to be protected against endogenous and exogenous factors that can have deleterious effects. As a consequence, it is highly desirable to facilitate and increase the amount of cell protection in the human body.

Poly-unsaturated fatty acids are most susceptible to lipid peroxidation, in particular the carbon(s) between two double bonds is susceptible to oxidation. Therefore mono-unsaturated fatty acids (MUFA's) are much less prone to oxidative damage than are poly-unsaturated fatty acids (PUFA's). It has surprisingly been found that changes in membrane acyl composition of both mitochondria and microsomes result in a decreased susceptibility of these membrane bilayers to lipid peroxidation.

Mono-unsaturated fatty acids, such as omega-7 and omega-4 fatty acids can function as an anti-oxidant and scavenge reactive oxygen species (ROS). By increasing the level of mono-unsaturated fatty acids in the cell membrane, cell peroxidation is decreased and thus cell protection and integrity is increased. As cells build up the several tissues of organisms, an increased amount of mono-unsaturated fatty acids confers whole organism protection, integrity and cell and tissue quality improvement.

Omega-4 fatty acids are mono-unsaturated fatty acids such as palmitolinoleic or hexadecadienoic acid (16:2(n-4)), hexadetreinoic acid (16:3(n-4)) or octadecatreinoic acid (18:3(n-4)).

Omega-7 (n-7) are a group of mono-unsaturated fatty acids including omega fatty acids such as palmitoleic acid (9-cis-Hexadecenoic acid; C16:1 (n-7)), hexadecadienoic acid (C16:2(n-7)), octadecadienoic acid (C18:2(n-7)), octadecadienoic acid (C18:3(n-7)) and eicosatrienoic acid (C20:3(n-7)). Palmitoleic acid (C16:1 n-7) is one of the eight important fatty acids which constitute over 95% of human milk. It is clear that nature has through evolutionary selection designed the best fatty acid composition possible for feeding human offspring. Other sources of palmitoleic acids are for instance fish or yeast. Palmitoleic acid (C16:1) in marine oils typically holds concentrations between 8-15% of the total fatty acid count. Naturally, palmitoleic acid derived from fishery produce is subject to seasonal fluctuations within species as well as differences between species. Literature indicates that this may vary more than tenfold.

In addition to the direct functions of palmitoleic (C16:1) acid (e.g. cell integrity, cell protection, cell hydration), this omega-7 fatty acid has further indirect beneficial functions. C16:1 being mono-unsaturated can be seen as a 'metabolically activated' C16:0 (saturated) which serves as precursor in the lipid metabolism. This metabolism yields numerous other fatty acids upon elongation, desaturation, etc. which have very important metabolic functions. This pathway can be wholly synthesized by the human/mammalian body. This is strongly different from the pathways of Essential Fatty Acids which cannot be synthesized from any precursors and on which there is a complete dependency on the diet. It is to be understood that C16:1 is a non essential fatty acid, which means that it can be synthesized from earlier precursors. This however does not mean that it is not important or even vital to proper functioning of the body.

By increasing the pool of 'metabolically activated' C16:1 the lipid pathways (the omega-7 series) are started further in the cycle and the downstream phases never run out of precursors and as such the entire pathway will function better and faster. We can see this as a sort of plentiful supply of 'vital precursors' through nutrition or topical application so as never to have a shortage of said vital precursors. In this way increased C16:1 improves the benefits derived from the downstream PUFA's e.g. 20:3 n-7 and the prostaglandins derived from this long chain fatty acids.

Prostaglandins belong to the group of eicosanoids and are hormones although rarely classified as such. They exert diverse actions on the cardiovascular, reproductive, respiratory, renal, endocrine, skin, nervous and immune systems. They regulate various essential physiological processes, amongst other but not limited to dilation resp. constriction of the vascular smooth muscle cells, aggregation or disaggregation of platelets, sensitization of spinal neurons to pain, decreasing intraocular pressure, regulate inflammatory mediation, regulate calcium movement (e.g. in osteoporosis respectively osteogenesis), control hormone regulation and control cell growth. They are very powerful but have a short half life before inactivation and excretion. This explains why a continuous supply of these prostaglandins is required and therefore a continuous and plentiful supply of the vital precursors.

Furthermore it has been shown that the direct precursor of the prostaglandins (i.e. 20:3 n-7) does not accumulate in animal tissues in significant amounts. Therefore it is self evident that it must be continuously derived from its own precursors, such as C16:1 n-7 which can accumulate in animal and human tissue. It is furthermore to be understood that C16:1 n-7 can through other pathways switch to for instance the omega-3 and/or omega-4 synthesizing pathways and sequences.

Through uptake of a composition comprising an omega fatty acid composition comprising one or more of a fatty acid selected from the group of omega-4 fatty acids and omega-7 fatty acids, **characterized in that** omega-4 and omega-7 fatty acids are present in at least 10% weight percentage of the TFAF of the composition, the cell membranes increases the protection of the cell against lipid peroxidation and improves thus the cell integrity and cell quality and regulation of the metabolic process through derivatives such as prostaglandins.

Preferably, the composition comprises omega-4 and omega-7 fatty acids in at least 30% weight percentage of the TFAF, and even more preferably, the composition comprises 50% weight percentage of the TFAF.

A composition providing an increased unsaturation in the acyl membrane composition of the cell is beneficial and provides improved functionality and increased protection against cellular damages. Increased concentrations of unsaturated fatty acids can be provided by an increase of both omega-4 and omega-7 fatty acids. Important to note is that the palmitolinoleic acid is synthesized from palmitoleic acid. Clearly they are interrelated in a metabolically significant way. Omega-4 and omega-7 fatty acids are balanced and interchangeable within the cell membrane.

In a preferred embodiment, consider the sum of the total percentage weight of the omega-4 and omega-7 fatty acids to be 100%. Those skilled in the art know that supercritical CO2 or fractionated distillation are exemplary processes available on the market place to obtain higher concentrations of selected omega fatty acids. The concentration of selected omega fatty acids obtained from such processes may be scaled up to 90% of weight of the TFAF and more. Tabel 1 shows a preferred embodiment of the present invention whereby omega-7 and omega-4 fatty acids are interrelated and interchangeable and the composition is designed so the sum of the total percentage weight of the omega-4 and omega-7 fatty acids is 100%.

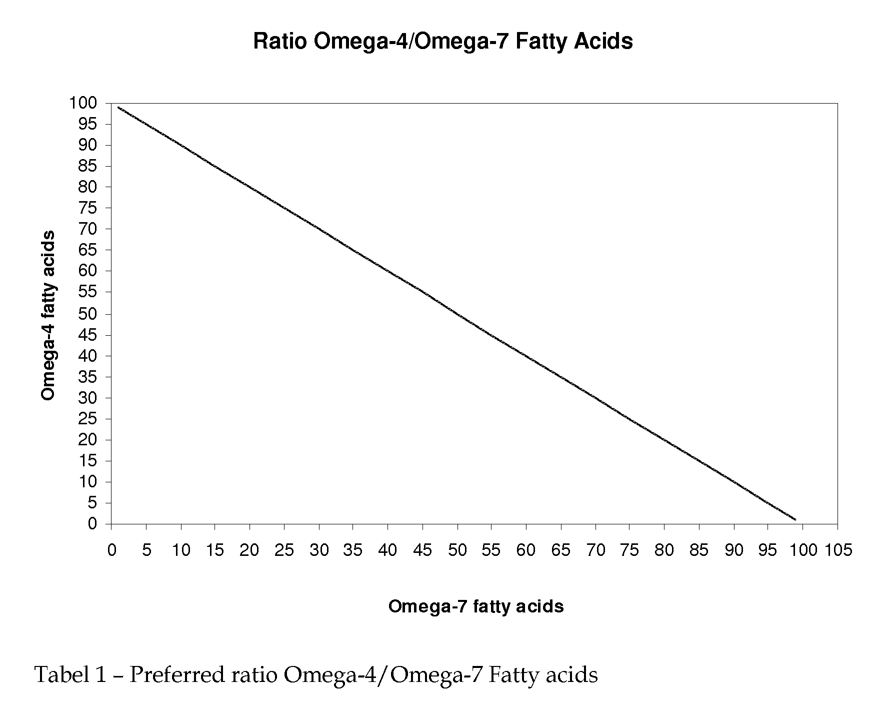

The fatty acids in this invention are to be considered in any form that they can occur, either free lipid form, esterified, phospholipid, triglyceride form etc. are understood to be included in this invention. The total fatty acid fraction (TFAF) is defined as comprising all aliphatic monocarboxylic acids derived from, or contained in esterified form in a vegetable, more preferably an algae fat, oil, or wax. Under fat, oil and/or wax is included all chemical compounds which is typically designated by the terms oils, fats and/or waxes.

In a preferred embodiment, the composition comprises structured triacylglycerols (TAG's). Basically a triacylglycerol is a backbone glycerol upon which three fatty acids are attached. These can be synthesized largely at will by techniques known to those skilled in the art. This enables the construction of TAG's with functional fatty acids, typically long chain fatty acids and short and or medium chain fatty acids which typically are lower in energy content. This results in low energetic high bio active TAG's. Many such products exist on the market. It is understood that algal C16:1 can be included in such structured TAG's. The same applies for structured phospholipids. The use of algal omega-7 and omega-4 fatty acids can find many applications such as but not limited to infant formulas, parenteral nutrition, nutritional beverages and functional foods and beverages, cosmetics, therapeutics and/or pharmaceutics.

Reshuffling of the triglyceride backbone of the fatty acids in the composition according to the present invention can be achieved by any number of methods known to those skilled in the art, such as enzymatic treatment. In the case of triglyceride ester derivatives, which when hydrolysed provide the unsaturated fatty acids, such as mono-unsaturated and poly-unsaturated fatty acids, which are the subject of the invention, all positional isomers of the unsaturated fatty acids substituents on the glycerol backbone are included. The triglycerides must contain at least one unsaturated fatty acid moiety. For example, of the three esterifiable positions on the glycerol backbone, the 1 and 2 positions may be esterified with mono- and/or poly-unsaturated fatty acids and by another lipid at position 3 or as an alternative, the glycerol backbone could be esterified by the fatty acid at the 1 and 3 positions with another lipid at position 2.

Preferably, the present invention is a composition extracted from microorganisms. Mass production of microorganisms, in particular algae, and more preferably diatoms, is possible in a continuous and sustainable way.

High concentrations of omega-4, omega-7 fatty and omega-3 acids are common in micro-algae. The group of Eustigmatophyceae, including the Nannochloropsis species, and particularly the group of Bacillariophyta (diatoms), have been found to contain high concentrations of omega-7, omega-4 and omega-3 fatty acids, as shown in Table 2.

**Tabel 2 - Exemplary and non limiting indicative overview of the fatty acid composition with a special emphasis on omega-4, omega-7 and omega-3. These fatty acids are commonly found in micro-algal oils harvested from micro-algae belonging to the groups Bacillariophyta (Diatoms).**

| | Bacillariophyta | | | Inferred range | |
|---|---|---|---|---|---|
| | MIN | AVERAGE | MAX | min | max |
| 16:1 N-7 | 13,8 | 27,4 | 36,3 | 10,0 | 40,0 |
| 18:1 N-7 | 0,4 | 0,9 | 1,9 | 0,1 | 3,0 |
| 16:2 N-7 | 0,9 | 4,3 | 28,4 | 0,1 | 40,0 |
| 16:2 N-4 | 0,7 | 2,7 | 4,7 | 0,1 | 10,0 |
| 16:3 N-4 | 1,0 | 6,6 | 22,0 | 0,1 | 40,0 |
| 18:2 N-4 | 0,0 | 0,2 | 2,0 | 0,0 | 10,0 |
| EPA | 6,7 | 17,9 | 26,6 | 3,0 | 50,0 |
| | | | | | |
| MUFA | 18,2 | 30,0 | 37,8 | 10,0 | 60,0 |
| PUFA | 18,5 | 38,0 | 62,9 | 10,0 | 80,0 |
| TO N-3 | 8,3 | 23,2 | 35,7 | 3,0 | 50,0 |
| TO N-7 | 14,5 | 28,2 | 37,3 | 5,0 | 50,0 |
| TO N-4 | 2,6 | 9,5 | 24,8 | 0,5 | 40,0 |
| | | | | | |
| TO 3+7 | 42,9 | 51,4 | 61,3 | 25,0 | 80,0 |
| TO 4+7 | 32,0 | 37,7 | 44,1 | 25,0 | 80,0 |
| TO 3+7+4 | 46,7 | 60,9 | 69,6 | 25,0 | 95,0 |
| | | | | | |
| RATIO 3/7 | 0,55 | 1,12 | 2,17 | 0,001 | 25,00 |
| | 0,24 | 0,72 | 1,82 | 0,001 | 25,00 |
| RATIO 4/7 | 0,22 | 0,50 | 1,65 | 0,001 | 10,00 |
| | 0,09 | 0,35 | 1,56 | 0,001 | 10,00 |
| RATIO 3/4 | 1,32 | 2,82 | 5,32 | 0,001 | 25,00 |
| | 1,17 | 3,42 | 6,35 | 0,001 | 25,00 |
| RATIO | | | | | |
| 3/4+7 | 0,42 | 0,74 | 1,10 | 0,001 | 25,00 |
| | | | | | |
| TO 3+7+4 | | | | | |
| % O 7 | 20,77 | 47,82 | 74,04 | 0,001 | 85,0 |
| % O 3 | 17,79 | 37,25 | 52,27 | 0,001 | 70,0 |
| % O 4 | 5,29 | 14,92 | 35,62 | 0,001 | 60,0 |

This is a non limiting indicative overview of the fatty acid composition of diatoms with special emphasis on omega-4, omega-7 and omega-3 fatty acids.

In Bacillariophyta (diatoms) the concentration of 03 + 04 + 07 ranges between 10 to 70, even 99% of the total fatty acid content. With the alga Nannochloropsis this could range from 30% upwards. The natural ratio 03/(07+04) in Bacillariophyta during the exponential growth phase ranges between 0,5 and 2,5, while during the exponential phase this ranges between 0,2 to 2,0. In Bacillariophyta the % of 07 + 04 of the summed concentration 03 + 04 + 07 ranges between 10-99% while conversely the % of 03 of 03 + 04 + 07 ranges between 1-70%.

Diatoms, such as those belonging to genera of the classes Coscinodiscophyceae, Fragilariophyceae or Bacillariphyceae, for instance but not limited to species belonging to the genera *Hydrosilicon, Thalassiosira, Melosira, Cyclotella, Stephanodiscus, Amphora, Nitzschia, Navicula, Pinnularia, Tabellaria, Fragilaria, Staurosira, Staurosirella, Aulacoseira, Skeletonema, Coscinondiscus, Odontella, Plagiogramma, Bellerochea, Chaetoceros, Diatoma, Martyana, Tabularia, Meridion, Licmophora, Eunotia, Actinella, Peronia, Cymbella, Encyonema, Gomphenema, Didymosphaenia, Phaeodactylum, Neidium, Sellaphora, Seminavis, Diploneis, Stauroneis, Fragilariopsis, Epithemia, Rhopalodia, Entomoneis, Surirella* and *Cymatopleura* are known to produce fatty acids. It has now surprisingly been found that the composition according to the present invention can be produced in and extracted from these species. The advantage of using algae, preferably diatoms as a source of omega fatty acids is that there is no dependency of seasonal fluctuations within species and differences between species observed in animal sources such as fish. Further, no complex regulations need to be followed because of using material derived from animals. An advantage of using algae as in stead of plant sources for omega fatty acids is that there is no geographical limitation and there is no seasonal dependency for collecting the harvest.

Further, microalgae are an innovative natural and vegetal omega fatty acids source with a high added value. Algae are a renewable resource and are optimal in nature preservation and to sustain an environmental friendly process. Microalgae are not only the main source of oxygen on Earth; they also absorb CO2 through photosynthesis. Microalgae cultures contribute to reduce atmospheric pollution. Another advantage of microalgae is that there is an economic benefit as the microalgae production process is ten times faster than for a plant.

Further, the present invention is also directed to a process for obtaining the composition described by the invention. The omega fatty acids are cultivated and stored in an algae biomass, preferably diatoms, either or not followed by extraction.

Another preferred embodiment of the present invention provides a composition further comprising other omega fatty acids such as omega-3, omega-6, omega-9, omega-11 and/or omega-13 fatty acids. The benefits of omega-3 and omega-6 fatty acids have already been suggested by many scientific studies, patent applications and patents, such as W002092073, wherein a combination of omega-3 and omega-6 fatty acids, more specifically a polar lipid-rich fraction containing gamma linolenic acid (GLA) and/or stearidonic acid (SDA) from seeds and microorganisms is used for human food applications, animal feed, pharmaceuticals and cosmetics.

Further, W002092540 provides a polar lipid-rich fraction containing eicosapentaenoic acid (EPA), docosahexaenoic acid (DHA), docosapentaenoic acid (PDA(n-3) ir DPA(n-6)), arachidonic acid (ARA), and eicosatetraenoic acid (C20:4n-3) from microorganisms, genetically modified seeds and marine organisms (including fish and squid) and their use in human food applications, animal feed, pharmaceutical and cosmetic applications.

Although the benefits of additional omega-3, omega-6, omega-9, omega-11 and omega-13 fatty acids has scientifically been proven, the effect in the long term on cell membrane lipid peroxidation is harmful as more of the sensitive and highly reactive poly-unsaturated fatty acids are incorporated in the cell membrane and hence, more production sites of ROS are available. In order to cope with this drawback and according to the present invention, omega-7 and omega-4 fatty acids are combined with these poly-unsaturated omega fatty acids to increase the level of protection offered to the cell membrane.

Omega-3 (n-3) is a group of poly-unsaturated fatty acids such as alpha linoleic acid (octadecatrienoic acid; C18:3(n-3);ALA), eicosapentaenoic acid (timnodonic acid; 20:5(n-3); EPA), docosapentaenoic acid (clupanodonic acid; 22:5(n-3); DPA) and docosahexaenoic acid (cervonic acid; 22:6(n-3) ;DHA), stearidonic acid (18:4(n-3);SA) and eicosatetraenoic acid (20:4(n-3);ETA). Omega-3 fatty acids are commonly extracted from fish oil, heterotrophic micro-organisms, seal blubber, algae and plants such as flax seed, sunflower, blackcurrant or borage.

An example of diatom micro-algal oil according to the present invention is extracted from the diatom Phaeodactylum tricornutum. This oil contains between 25-35% palmitoleic acid. Peaks of over 40% have been observed in certain treatments. 7-8% omega-4 fatty acids, such as C16:2(n-4) has been observed in Phaeodactylum tricornutum. In addition to this omega-7 fatty acid, the micro-algal oil extracted from Phaeodactylum tricornutum contains up to 30% omega-3 fatty acid eicosapentaenoic acid (EPA). The combined presence of these tree fatty acids makes this algal oil very valuable.

It has been shown that functional fatty acids yield a higher bio-activity when used in conjunction with certain other bio-activity exhibiting compounds. Thus, by combining the omega-4 and omega-7 fatty acids with other fatty acids, such as omega-3, omega-6 and/or omega-6 fatty acids, a lower concentration active component is needed by the synergistic, cumulative effect of the different active compounds.

There is a large body of scientific evidence to assume that a number of the activities are amplified by the combination with omega-7 and omega-4 fatty acids. Especially, but not limited to, in those conditions where cellular membranes, skin, lipid integration and permeability are concerned. The composition according to the present invention increases the immune response activity as well.

In a preferred embodiment, the ratio of omega-3 to omega-4 and omega-7 fatty acids ranges from 1:5 to 5:1, preferably 1:3 to 3:1 and even more preferably 1:2 to 2:1.

As the composition according to the present invention is beneficial for all cells and all tissues in the human and/or animal body, there are many disorders that can be treated with the composition provided in the invention. The composition restores imbalances in the saturated /mono-unsaturated/poly-unsaturated lipid profile of the cell membrane and increases the level of mono-unsaturated lipids, whereby lipid peroxidation is controlled and decreased. The present invention is directed to the role of dietary fatty acid profile on membrane composition and, in turn, its nutritional, therapeutic and cosmetic effect on the human and animal body. The present invention is further directed to a topical and/or oral product, with a special focus on nutrients, functional food and feed, therapeutic and cosmetic products. A nutrient is a chemical that an organism needs to live and grow or a substance used in an organism's metabolism which must be taken in from its environment. The nutrient is an essential nutrient to an organism if it cannot be synthesized by the organism in sufficient quantities and must be obtained from an external source.

The long-chain poly-unsaturated fatty acids are well known for their nutritional importance. They have been shown to be vital for brain development, beneficial for the cardiovascular, nervous, gastro-intestinal, musculoskeletal, metabolic, respiratory and/or uro-genital system and for other important nutraceutical and pharmaceutical targets in human and animal health. Unsaturated fatty acid compositions can further be used for treating allergy disorders, inflammation or immunologic disorders and/or tumors. They are also used in dermatologic and/or cosmetic products.

The topical and/or oral composition according to the present invention may provide the fatty acid composition of the cell membrane with an additional amount of omega-4 and omega-7 fatty acids. A balanced shift in the ratios saturated fatty acids/mono-unsaturated fatty acids/poly-unsaturated fatty acids may be beneficial to make the cell membrane optimally resistant against peroxidase and cell ageing.

### CARDIOVASCULAR SYSTEM

Scientific studies give proof of the structural function of palmitoleic acid in arterial walls. As the long-chain poly-unsaturates in membrane bilayers of mitochondria are very susceptible to damage, membrane lipid peroxidation and many of its products which are themselves very potent damagers of proteins are thus a serious threat for the arterial cells.

Several cardiovascular diseases may be the result of disorders in the arterial cell membrane, such as atherosclerosis, thrombosis, cortisol insensitivation, endothelial damage, cardiovascular disease, myocardial infarction, modulation of atherogenesis, regulation of blood pressure and hypertension, hypercholesterolemic effect, increased HDL cholesterol, angiopathy, lowering serum cholesterol and LDL, ventricular arrhytmics and metabolic syndrome.

Diets rich in mono-unsaturated cis-fatty acids are associated with a significant reduction of cardiovascular risk. It has been shown that upon their incorporation into the plasma membrane, cis-fatty acids induce a marked perturbation of the lipid domains, altering membrane fluidity as well as lipid-lipid and lipid-protein interactions in the bilayer plane.

Many of the experimentally induced forms of atherosclerosis are dependent on endothelial injury and subsequent platelet-macrophage-endothelium interactions. This is the 'response to injury hypothesis', proposed by Ross. It is the primary cause for the development of atherosclerosis. The endothelial injury may be subtle or intermediate or ultimately result in cell death. It has been demonstrated that platelet and macrophage interactions with the endothelium are required for lesion formation. These lesions may manifest themselves as endothelial cell separation from connective tissue, endothelial cell death, and/or increased endothelial cell permeability; any of these may result in endothelial retraction and endothelial cell desquamation. Many of these damaging events involve ROS. injury to the endothelial cells can result from exposure to amongst others lipid peroxides, hypercholesterolemia and/or hyperlipaemia.

Studies show that palmitoleic acid and HDL-cholesterol are significant independent predictors of endothelial function. Palmitic/palmitoleic acid and stearic/oleic acid is heterogeneously distributed over the aorta with high concentration areas located especially in the tunica media region of the aorta. Human atherosclerotic plaque show an irregular cholesterol distribution mainly located in spots in the intima region with elongated diacylglycerol regions located mainly in the media region. As palmitoleic acid is positively associated with endothelial functioning, while reduced endothelial functioning (EDV) is an early marker for atherosclerosis, it is clear that it has an antioxidant capacity protecting against free radicals and dangerous compounds resulting from lipid peroxidation.

A higher concentration of mono-unsaturated fatty acids provided by a composition according to the present invention in arterial cell membranes may have a protective effect on vasodilation in general. Hence, an omega fatty acid composition comprising one or more of a fatty acid selected from the group of omega-4 fatty acids and omega-7 fatty acids, **characterized in that** omega-7 and omega-4 fatty acids are present in at least 10% weight percentage of the TFAF of the composition. Uptake of this composition by the cell membranes increases the protection of the cell against lipid peroxidation and improves thus the cell integrity and quality.

### NERVOUS SYSTEM

Nutritional studies show that ageing in animals can be significantly influenced by dietary restriction. This may open up new perspectives in medicine, as molecules inducing this defense mechanism, such as mono-unsaturated fatty acids may be possible candidates for novel cytoprotective strategies. In particular, manipulation of endogenous cellular defense mechanisms, such as the heat shock response, through nutritional anti-oxidative and pharmacological compounds, may represent an innovative approach to therapeutic intervention in diseases causing tissue damage, such as neurodegeneration.

The membranes of neurons, the specialized brain cells that communicate with each other, are composed of a thin double-layer of fatty acid molecules. Myelin is the protective sheath that covers communicating neurons. It is a very complex substance, consisting of various fats, fatty acids, phospholipids, proteins and even cholesterol. The vast majority of these substances are either fat or fat derivatives. It is therefore clear that the formation and periodic regeneration of myelin depends on dietary intake and a well-functioning metabolic system.

The myelin sheet is composed of 30% protein and 70% fat. The myelin sheath serves as insulation against signal loss to neighboring tissue. A damaged myelin sheath leads to a distortion or complete disappearance of signals. Many neurologic disorders, such as multiple sclerosis, are caused by damage to the myelin sheath.

Oxidative stress and mitochondrial dysfunction have been closely associated in many subcellular, cellular, animal, and human studies of both acute brain injury and neurodegenerative diseases. There is significant evidence that the pathogenesis of several neurodegenerative diseases, including Parkinson's disease, Alzheimer's disease (AD), Friedreich's ataxia (FRDA), multiple sclerosis and amyotrophic lateral sclerosis, may involve the generation of reactive oxygen species (ROS) and/or reactive nitrogen species (RNS) associated with mitochondrial dysfunction.

Evidence for mitochondria being a site of damage in neurodegenerative disorders is based in part on observed decreases in the respiratory chain complex activities in Parkinson's, Alzheimer's, and Huntington's disease. A defective mitochondrial respiratory chain, associated with increased free radical generation and oxidative damage, may be considered possible mechanisms that compromise cell viability. Several conditions including protein, lipid or glucose oxidation disrupt redox homeostasis and lead to accumulation of unfolded or misfolded proteins in the aging brain. Alzheimer's and Parkinson's diseases or Friedreich ataxia are neurological diseases sharing, as a common denominator, production of abnormal proteins, mitochondrial dysfunction and oxidative stress, which contribute to the pathogenesis of these so called "protein conformational diseases".

Alzheimer's disease is the most common form of neurodegenerative disorder with dementia. In its sporadic form, AD results from the combination of genetic factors with different epigenetic events. Among them, oxidative metabolic reactions and their by-products have been consistently implicated in AD pathogenesis and represent the biological basis for the 'oxidative stress hypothesis' of AD. Numerous studies demonstrate that different biomarkers of oxidative-stress-mediated events are elevated in the AD brain. Oxidative stress, a hallmark of Alzheimer disease (AD), has been shown to induce lipid peroxidation and apoptosis disrupting cellular homeostasis.

Because RNA is mostly single-stranded and its bases are not protected by hydrogen bonding and are less protected by specific proteins, RNA may be more susceptible to oxidative insults than DNA. Such non-acutely lethal insults to cells might be associated with underlying mechanisms of several human diseases, especially chronic degeneration. Recently, oxidative RNA damage has been described in several neurodegenerative diseases including Alzheimer disease, Parkinson disease, dementia with Lewy bodies, and prion diseases. Of particular interest, oxidative RNA damage is a feature in vulnerable neurons at the very earliest-stages of these diseases, suggesting that RNA oxidation may actively contribute to the onset or to the development of disease. The detrimental effects of oxidative damage to protein synthesis may be reduced, at least in part, by the composition according to the invention which provides a therapeutic strategy for neurodegenerative and other degenerative diseases.

Insulin and cholesterol play important roles in basic metabolic processes in peripheral tissues. There is a pool of cholesterol in the brain from which all metabolizing structures, including myelin, draw their cholesterol supplies. There is continuous exchange of cholesterol between the brain pool and the blood. The rate of this exchange may be related to the rate of blood flow through the tissue. Both insulin and cholesterol can also act as signaling molecules in the central nervous system that participate in neuronal function, memory and neurodegenerative diseases.

Many of the unexplained features of Alzheimer's disease (AD), such as cell death and tangles in the brain, appear to be linked to abnormalities in insulin signaling. Insulin disappears early and dramatically in AD patients, suggesting links between AD and diabetes.

Palmitoleic acid protects the cells against cell death and increases insulin sensitivity. Further, the mediation of palmitoleic acid on blood cholesterol is important for various neuropathologies, such as Alzheimer's disease and multiple sclerosis. Thus, an omega fatty acid composition comprising one or more of a fatty acid selected from the group of omega-4 fatty acids and omega-7 fatty acids, **characterized in that** omega-7 and omega-4 fatty acids are present in at least 10% weight percentage of the TFAF of the composition, either or not combined with other omega fatty acids may have very beneficial properties. Through its mediating/stabilizing part in cholesterol, one of the most important molecules for neurons and the brain, there are additional benefits for the brain and indeed so for Alzheimer.

Also, as palmitoleic acid is one of the more important fatty acids of nervous cell membranes and being specific precursor for a number of proteins, such as phosphocholine glyceride transferase, it is being considered a vital fatty acid for the brain, nervous system and its proper functioning.

Another neurologic disorder is attention deficit hyperactivity disorder (ADHD). ADHD comprises a range of behavioral problems including inattention, hyperactivity and impulsivity. Several studies have identified abnormalities in membrane fatty acids in some subjects with ADHD, and success has been reported using lipid therapies. Scientific data suggests that some patients with ADHD have higher rates of oxidative breakdown of n-3 poly-unsaturated fatty acids (PUFA's). Such a biochemical abnormality may underlie the previously observed fatty acid deficiencies, as well as providing further rationale for the use of anti-oxidant and/or lipid supplementation therapy in the treatment of ADHD.

Major depression is another important neurologic disorder. It is characterized by symptoms at the psychological, behavioral and physiological levels. In depressive patients, changes in fatty acid concentrations were found. One of the most frequent symptoms of depression is sleep disturbances. Palmitoleic and eicosadienoic acids have the strongest connections with sleep performance. Palmitoleic and oleic acid seem to be especially important for sleep disorders, may be due to their function as precursors of the sleep inducing oleamide. Linoleic and eicosadienoic acid could be helpful for maintaining sleep because they are precursors of the sleep mediator PGD2. There is a significant lack of omega-3 fatty acids, special monoenoic and omega-6 fatty acids in sleep-disturbed depressives. High concentrations of palmitoleic acid and lauric acid, a saturated fatty acid, were related to a low level of depression (BDI and Hamilton scores). Increases in oxidant production and lipid peroxidation level in brain regions can be overcome through simultaneous treatment with the composition according to the invention.

Symptoms associated with neurodegeneration include both physiological and biological symptoms including, but not limited to: neurodegeneration, intellectual decline, behavioral disorders, sleep disorders, common medical complications, dementia, memory loss, psychosis, anxiety, depression, manic depression, mood conditions, migraine soothing, borderline, inflammation, pain, and dysphagia, and disorders such as bipolar disorder, dyslexia, dyspraxia, attention deficit hyperactivity disorder (ADHD), attention deficit disorder (ADD), epilepsy, autism, Alzheimer's Disease, Parkinson's Disease, senile dementia, peroxisomal proliferator activation disorder (PPAR), multiple sclerosis, diabetes-induced neuropathy, macular degeneration, retinopathy of prematurity, Huntington's Disease, amyotrophic lateral sclerosis (ALS), retinitis pigmentosa, cerebral palsy, muscular dystrophy, cancer, cystic fibrosis, neural tube defects, depression, Zellweger syndrome, Lissencephaly, Down's Syndrome, Age-Related Macular Degeneration (ARMD), Muscle-Eye-Brain Disease, Walker- Warburg Syndrome, Charoct-Marie-Tooth Disease, inclusion body myositis (IBM) and Aniridia are all known to be disorders of the nervous system.

The objective of the composition according to the present invention is to cure the sufferer or at least reduce the symptoms to bearable levels by preventing lipid peroxidation of the nervous cell membranes. It is a further objective to provide a nutritional or pharmaceutical composition for neurotransmission, mental performance and neurological disorders such as Alzheimer's disease, depression, schizophrenia, attention-deficit hyperactivity disorder, and protection of the cells against development of neuronal dysfunction and offer additional functional fatty acids to the cells that are vital in the maintenance of normal brain and nervous system function.

### HUMAN DEVELOPMENT

Fatty acids can influence important cellular and hormonal processes in the human body. Non-adequate contents of fatty acids, e.g., in blood, can cause and/or result in various diseases.

Infant food is already supplied with omega supplements, as shown in US5539133, GR3001950T and WO96/21037, providing omega-3 and omega-6 fatty acid mixtures as additive for infant formulas. For the development of all the infant's organs, it is of utmost importance that the milk and diet is rich of omega fatty acids. Palmitoleic acid (C16:1 n-7) is one of the eight important fatty acids which constitute over 95% of human milk. It is clear that nature has through evolutionary selection designed the best fatty acid composition possible for feeding human offspring.

The eye, more particular, the interphotoreceptor matrix contains relatively high concentrations of endogenous fatty acids (palmitic (35%), stearic (21 %), palmitoleic (7%), oleic (29%), linoleic (6%) and docosahexaenoic acids (2%). These fatty acids account for about 90% of the total fatty acids bound to interphotoreceptor matrix proteins extracted with organic solvents. Specific proteins may depend on the covalently bound fatty acids for anchoring in the outer leaflet of cell membranes. Hence, it is important to provide the infant with sufficient levels of omega fatty acids. As the composition according to the present invention comprises omega-7 fatty acids, such as palmitoleic acid, combined with omega-4 and preferably other omega fatty acids, such as the omega-3 fatty acid docosahexaenoic acid, it is beneficial for the development of the infant's cells, tissues, organs and whole body.

The membrane composition is not only important for the developing child, it is important during the whole lifespan of an organism. Membrane bilayer composition may have a role in determining this maximum lifespan (MLSP). Whilst the physical properties imparted to membranes by their acyl chains influence metabolic rate, it is their chemical properties that are responsible for their role in aging. Via their role in lipid peroxidation, membrane acyl composition is related to maximum lifespan in mammals and birds. The acyl composition of the diet further significantly alters the metabolic rate. The 'oxidative stress theory' hypothesizes that death from old age is that it is the result of accumulated damage from reactive oxygen species (ROS) that are an inevitable byproduct of mitochondrial oxygen consumption.

Oxygen-derived free radicals are responsible for the age-related damage at the cellular and tissue levels. In a normal situation, a balanced-equilibrium exists among oxidants, antioxidants and biomolecules. Excess generation of free radicals may overwhelm natural cellular antioxidant defences leading to oxidation and further contributing to cellular functional impairment. The identification of free radical reactions as promoters of the aging process implies that interventions aimed at limiting or inhibiting them should be able to reduce the rate of formation of aging changes with a consequent reduction of the aging rate and disease pathogenesis.

Antioxidant supplementation, more particular, the composition according to the present invention supplied in a topical and/or 'per os' application, might improve major outcomes of interest in humans and animals. Physical performance, muscle strength, overall longevity and reduction of clinical conditions related to for instance the gastrointestinal system, the eyes, the skin, the heart, the teeth, the cardiovascular system, multiorgan failure, the cerebral system and the respiratory system is stimulated by increasing the level of mono-unsaturated fatty acids in the cell membrane and thus increasing cell protection and integrity.

### INFLAMMATION

Dietary lipids have the capability to modify immune function. The three major mechanisms include alterations in eicosanoid synthesis, changes in lipoprotein levels and modifications in membrane composition which affect transmembrane signal transduction. The same goes for topical applications which enter tissues and are metabolized there.

Atherosclerosis is a disease of the vessel wall that involves lipid accumulation, chronic inflammation, cell death, and thrombosis, all these eventually leading to heart disease and/or stroke. Inflammation and oxidative stress are key factors in atherogenesis contributing significantly to the initiation, progression, and rupture of lipid-rich atherosclerotic plaques. Associated with the inflammatory response and oxidative stress are lipid peroxidation and formation of bioactive lipids. Bioactive lipids include amongst others oxidized free fatty acids and oxidized phospholipids (oxPL).

Bioactive lipid peroxidation products accumulate during viral infections and in inflammatory conditions such as rheumatoid arthritis and atherosclerosis and are also generated in apoptotic and necrotic cells. Specifically, oxidized phospholipids are generated when low density lipoprotein (LDL) or cellular phospholipids containing poly-unsaturated fatty acids (PUFA's) at the sn-2 position undergo oxidative attack. A common consequence of such attack is oxidative fragmentation of the sn-2 residue with generation of phospholipid molecular species containing shortened sn-2 residues.

Accumulation of oxidative modified proteins in cellular targets and tissues occurs during aging, oxidative stress, and in a variety of diseases including atherosclerosis. 4-Hydroxy-2-alkenals are prominent aldehyde substances generated during the peroxidation of PUFA's, such as omega-6 PUFA's (e.g. linoleic and arachidonic acid) and omega-3 PUFA's (e.g. docosahexaenoic and eicosapentaenoic acid).

Lipid oxidation products thus accumulate in inflamed and oxidative damaged tissue, where they are derived from oxidative modification of lipoproteins, but also from membranes of cells undergoing apoptosis. Oxidative phospholipids have a central role in disease states associated with dyslipedemia, including atherosclerosis, diabetes and its complications, metabolic syndrome, and renal insufficiency, as well as general prothrombotic states. In addition, in organs which are constantly exposed to oxidative stress, including lung, skin, and eyes, increased levels of oxPL are suggested to contribute to inflammatory conditions. Moreover, accumulation of oxPL causes general immunomodulation and may lead to autoimmune diseases. Evidence is accumulating that oxPL play a role in lupus erythematosus, antiphospholipid syndrome, rheumatoid arthritis and psoriatic arthritis. Last but not least, a role for oxPL in neurological disorders including multiple sclerosis (MS), Alzheimer's and Parkinson's disease has been suggested.

Mono-unsaturated fatty acids, such as omega-7 and omega-4 fatty acids can function as an anti-oxidant and scavenge reactive oxygen species (ROS). By increasing the level of mono-unsaturated fatty acids in the cell membrane, cell peroxidation is decreased and thus cell protection and integrity is increased. As cells built up the several tissues of organisms, an increased amount of mono-unsaturated fatty acids confers whole organism protection, integrity and cell and tissue quality improvement. A way to prevent and/or decrease lipid peroxidation is by the composition according to the present invention.

### SKIN

Omega-7 is an unsaturated fatty acid that is present in the phospholipid bilayer of the cell membrane. Hence, it is present in all tissues of the human body. For example, it is a natural component of skin, human largest organ, playing an important role in skin physiology. Omega-7 has been shown to help nourish skin and help skin maintain its normal cell structure. Delicate body tissues, like skin and membranes that line the digestive and urogenital tracts, have the greatest affinity for omega-7 and/or omega-4 fatty acids.

Environmental stressors (such as sunlight and pollution), improper foods, and even normal aging can challenge those sensitive membranes, leading to inflammation process. Consequences may be unaesthetic and painful. It has now been found that an increase of mono-unsaturated omega fatty acids in the cell membrane can lead to a decrease in cell peroxidation. Mono-unsaturated fatty acids, in particular, omega-7 fatty acids, are now being hailed as an agent to protect, replenish, moisturize, and restore.

According to a further aspect of the present invention there is provided a composition comprising at least one omega-7 and at least one omega-4 fatty acid, for providing at least one skin care benefit selected from: treating/preventing wrinkling, sagging, aged and/or photo-damaged skin; boosting collagen deposition in skin, boosting decorin production in skin, enhancing tissue repair; lightening skin; improving skin condition and resilience through enhanced barrier formation; treating dry and flaky skin; soothing irritated, red and/or sensitive skin; and improving skin texture, smoothness and/or firmness; the composition being in the form of a topical or an oral product for applying to the skin.

Scientific research has showed that the omega-4 and omega-7 mono-unsaturated fatty acids, such as palmitoleic acid (C16:1 n-7) and the fatty acid 6,9,12-hexadecatrienoic acid (HTA, C16:3 n-4) are active against Gram-positive bacteria. Further, it has been shown that palmitoleic acid is active at micro-molar concentrations, kills bacteria rapidly, and is highly active against multidrug-resistant Staphylococcus aureus (MRSA). MRSA isolates are an emerging cause of infections, especially skin and soft tissue infections. Staphylococcus aureus is further the leading etiological agent infecting skin of the patients with atopic dermatitis and eczema.

The composition according to the present invention may have a dermatological effect, both cosmetic and therapeutic. By providing the cells of the skin with an additional amount of mono-unsaturated fatty acids, the cells are not only hydrated, but they are also provided with an additional defense against ageing from cell oxidation and additionally protected against diseases such as psoriasis, eczema, atopic eczema, skin erythema, acne, photodermatitis, sun allergies and stretch marks. The composition is anti-inflammatoir. Further, the composition provides a stabilization of the cell membrane structure, cell regeneration, tissue hydration, improves skin elasticity and skin firmness and may have an anti-wrinkle and anti-ageing effect.

Scientists now know that omega-7 unsaturated fatty acids found on the skin surface were oxidatively decomposed to 2-nonenal, which may be the cause of the phenomenon commonly known as old person smell. This makes it possible to manipulate the composition and design young people, teenager, babysmell fragrances and cosmetics.

The cosmetic methods and the uses of the unsaturated fatty acids and/or derivatives according to the invention may be useful for treating skin which is already in a wrinkled, aged, photodamaged and/or irritated condition or for treating youthful skin to prevent or reduce those aforementioned deteriorative changes due to the normal aging/photoaging process. It generally enhances the quality of skin and improves its appearance and texture by preventing or reducing wrinkling and increasing flexibility, firmness, smoothness, suppleness and elasticity of the skin and skin lightening.

The composition used according to the invention also comprises a dermatologically/cosmetically acceptable vehicle to act as a dilutant, dispersant or carrier for the active highly unsaturated fatty acid or its derivative. The vehicle may comprise materials commonly employed in skin care products such as water, liquid or solid emollients, silicone oils, emulsifiers, solvents, humectants, thickeners, powders, propellants and the like. Supplements such liquid silica, biotin, CoQlO and hyaluronic acid can be combined with the composition according to the present invention in a unique 100% vegetarian product.

Besides the highly unsaturated fatty acid active, other specific skin-benefit actives such as sunscreens, other skin lightening agents, and skin tanning agents may also be included. The vehicle may also further include adjuncts such as perfumes, opacifiers, preservatives, colourants and buffers. Other preferred adjuncts include other known skin care benefit agents, moisturisation agents, agents known to improve skin condition, and especially antioxidants, such as BHT, tocopherol, ascorbyl acetate, quercetins, phytosterols and green tea polyphenols.

The composition can further be used in after sun products, in after shaves and depilatories, in babycare products and in tooth paste and mouth washes.

### GASTRO-INTESTINAL SYSTEM

The digestive tract is the system of organs within multicellular animals that takes in food, digests it to extract energy and nutrients, and expels the remaining waste. The mucosa is the innermost layer of the gastrointestinal tract that is surrounding the lumen, or space within the tube. This layer comes in direct contact with the food (or bolus), and is responsible for absorption and secretion, important processes in digestion.

Healthy and strong mucous membranes are important for general health and well being. Mucus membranes, as any other membranes are dependent of the relative balance between mono-unsaturated and long-chain poly-unsaturated acyl chains in membrane bilayers which is a fundamental determinant of metabolic rate of a species. It provides a framework to understand factors such as the influence of diet on metabolism.

A healthy diet favorably modifies the membrane lipid profile. Several clinical conditions of the gastro-intestinal system, such as ulcerative colitis (UC), Crohn's disease (CD) and inflammatory bowel disease (IBD) have been linked to oxidative stress damages. To protect the cell against the result of accumulated damage from ROS, the acyl composition need saturated and mono-unsaturated acyl chains lacking the carbon atoms between the -C=C- units found in poly-unsaturated acyl chains and which are most susceptible to oxidative attack. Mono-unsaturated fatty acids, such as C16:1 can function as an anti-oxidant and scavenge ROS.

Intestinal inflammation is accompanied by excessive production of reactive oxygen and nitrogen metabolites. In order to counteract their harmful effects, the intestinal mucosa contains an extensive system of antioxidants. It has been shown that the levels of and the balance between the most important antioxidants are seriously impaired within the intestinal mucosa from inflammatory bowel disease (IBD) patients compared with normal mucosa. The knowledge of importance of oxidative stress in the pathogenesis of IBD, CD, UC and other gastro-intestinal diseases, provides clues regarding the (anti)oxidants involved which indicate that omega fatty acids can be used as supplement to prevent, control and/or decrease oxidative stress.

Oxidative damage may also result in metabolic syndrome or other gastrointestinal conditions such as diarrhea, rapid intestinal transit, dumping syndrom, weight loss, distention, steatorrhea, malnutrition, reflux, gastric aphthous and/or peptic ulcers, postgastrectomy syndrom, short bowel syndrom, gastrointestinal peptide tumors, abdominal pain, abdominal cramping, cystic fibrosis, diseases of the mucous membrane, oesophagitis and combinations thereof.

A way to prevent and/or decrease lipid peroxidation is by the composition according to the present invention, the composition comprising mono-unsaturated fatty acids, such as omega-7 and omega-4 fatty acids, which can function as an anti-oxidant and scavenge reactive oxygen species (ROS).

### METABOLIC SYSTEM

Mono-unsaturated fatty acids form a very important platform in numerous metabolical pathways. The presence of fat in the small intestine produces hormones which stimulate the release of lipase from the pancreas and bile from the gallbladder which breaks down the fat into monoglycerides and fatty acids. The bile emulsifies the fatty acids so they may be easily absorbed. Short- and some medium chain fatty acids are absorbed directly into the blood via intestine capillaries and travel through the portal vein just as other absorbed nutrients do. However, long chain fatty acids and some medium chain fatty acids are too large to be directly released into the tiny intestinal capillaries. Instead they are absorbed into the fatty walls of the intestine villi and reassembled again into triglycerides. Cholesterol plays an essential role in the digestion of dietary fats. The triglycerides are coated with cholesterol and protein (protein coat) into a compound called a chylomicron, which transport the triglycerides to where they are needed.

Scientists have found that a diet high in PUFA's modifies the acyl composition of membrane bilayers and is associated with increased membrane lipid peroxidation.

Metabolic syndrome is a combination of medical disorders that increase the risk of developing cardiovascular disease and diabetes. It affects one in five people, and prevalence increases with age. Metabolic syndrome is also known as insuline-resistance syndrome.

Further, a high intake of fat may increase the risk of obesity. Obesity, especially abdominal obesity, is an important determinant of the risk of developing insulin resistance and non-insulin-dependent diabetes mellitus.

Research has shown that a diet enriched with mono-unsaturated fatty acids may facilitate uptake and utilization of glucose in normal and insulin-resistant skeletal muscle cells. Thus, omega-7 and omega-4 fatty acids are beneficial in metabolic syndrome and obesity where insulin resistance is a major problem.

Macadamia nut consumption was associated with a significant increase in the relative intake of MUFA's and a reduced relative intake of saturated fatty acids and PUFA's. Scientific studies demonstrate that macadamia nuts, known for their high omega-7 fatty acid content, as part of a healthy diet favorably modifies the plasma lipid profile in hypercholesterolemic men despite their diet being high in fat.

Increased LDL/cholesterol reduces total mortality (heart disease and others), reduces ischaemic and haemorrhagic strokes, etc. in general metabolic syndrome. Scientific research shows that cholesterol levels may be stabilized, for instance through dietary intake of palmitoleic acid.

Metabolic diseases such as chronic fatigue syndrome (CFS), metabolic syndrome, diabetes, diabetes type III (Alzheimer), insulin resistance, cortisol insensitivation, obesitas, hypercholesterolemia, pancreatitis may be stabilized, controlled and/or prevented by supplementary intake of the composition according to the present invention.

### RESPIRATORY SYSTEM

It is generally acknowledged that anti-oxidation is a very essential step to whole body health. By example, healthy and strong mucous membranes are important for general health and well being. Mucus membranes are surface epithelial tissues that line cavities exposed to the outside environment, such as most of the respiratory system.

Gastroesophageal reflux has been suggested as an underlying cause of chronic lung disease. Scientific research has reported that protein oxidation in bronchoalveolar lavage fluid is higher in children with extensive proximal acidic reflux, suggesting that pulmonary microaspirations contribute to lung damage. Patients' fatty acid profiles depended on the pulmonary function and the inflammation state. These results suggest that dietary fatty acids may play a role in lung disease such as chronic hyperventilation syndrome, cystic fibrosis and pneumoconiosis.

The increased occurrence of respiratorial allergies and asthmatic conditions in young children and increasingly also in adults, in our Western society is often attributed to overly polluted air conditions. Many triggers can cause allergies or asthma, which sometimes remain single events but sometimes develop into long term inflammatory conditions. Such conditions of the respiratory system may be positively affected or countered by a dietary supplementation with omega 7 and omega-4 fatty acids. There is scientific evidence to infer that these fatty acids help protect the airways. This may in part be through fortifying and repairing the reportorial mucous membranes.

Further, omega-3 fatty acids are known to have strong anti-inflammatory activities. As asthma can take on a long term inflammation condition of the reportorial system it stands to reason that omega-3 fatty acid supplementation will be beneficial to reducing inflammation of said reportorial tissues and help alleviate the adverse condition of the afflicted person

The combined effect of fortifying and repairing the afflicted reportorial tissues by the composition according to the present invention, comprising omega-7 and omega-4 fatty acids, and optionally the anti-inflammatory (both preventive and curative) properties of omega-3 fatty acids are stronger than the individual activities. This synergistic effect is very beneficial for the afflicted person.

### MUSCULOSKELETAL SYSTEM

Several studies have shown that the fatty acid composition of the phosholipids of the skeletal muscle cell membranes is closely related to insulin sensitivity. An increased saturation of the membrane fatty acids and a reduced activity of delta 5 desaturase have been associated with insulin resistance. There are several possible mechanisms which could explain this relationship. The fatty acid composition of the lipids in serum and muscle may, amongst others, be influenced by diet. A diet enriched with mono-unsaturated fatty acids may facilitate uptake and utilization of glucose in normal and insulin-resistant skeletal muscle.

The composition according to the present invention, comprising omega fatty acids selected from omega-4 and omega-7 fatty acids might be beneficial for musculoskeletal disorders such as arthritis, osteoporosis, gout, carpal tunnel syndrome, rheumatoid arthritis and diabetes related musculoskeletal diseases.

### ANTI-BIOTIC ACTIVITY

Scientific studies have shown that fatty acid extracts marked by EPA, an omega-3 fatty acid, contain further antibacterial compounds. The antibacterial compounds were identified as the mono-unsaturated omega-7 fatty acid (9Z)-hexadecenoic acid (palmitoleic acid; C16:1 n-7) and the omega-4 fatty acid (6Z, 9Z, 12Z)-hexadecatrienoic acid (HTA; C16:3 n-4). Both are active against Gram-positive bacteria with HTA further inhibitory to the growth of the Gram-negative marine pathogen, Listonella anguillarum.

Palmitoleic acid is active at micro-molar concentrations, kills bacteria rapidly, and is highly active against multidrug-resistant Staphylococcus aureus (MRSA in pigs or other organisms, VRSA). An isomer of palmitoleic acid in human skin sebum is effective against gram-positive bacteria, which has possible applications in foot care.

The composition according to the present invention could find application in the topical and systemic treatment of drug-resistant bacterial infections, protecting and restoring at tissue and cellular level for instance through cell membranes and reducing the adverse effects of bacterial, fungal, yeast activities

In a further embodiment according to the present invention, the composition comprising omega-7 and omega-4 fatty acids is combined with other active components, such as omega-3 fatty acids. Omega-3 fatty acids have anti-inflammatory and anti-proliferative actions and may further be important in reducing the risk of benign conditions

### ANTI-TOXIC ACTIVITY

The cumulative production of ROS and reactive nitrogen species (RNS) through either endogenous or exogenous insults is termed oxidative stress and is common for many types of cancer cell that are linked with altered redox regulation of cellular signalling pathways. Oxidative stress induces a cellular redox imbalance which has been found to be present in various cancer cells compared with normal cells; the redox imbalance thus may be related to oncogenic stimulation.

The ratio of saturated to monounsaturated fatty acids affects phospholipid composition and alteration in this ratio has been implicated in a variety of disease states including cardiovascular disease, obesity, diabetes, neurological disease, and cancer. By contrast, mono-unsaturated fatty acids (e.g. palmitoleate) are not toxic and can protect against the detrimental effects of palmitate. Mono-unsaturated fatty acids control cell viability by regulating a different step in the apoptotic pathway from that influenced by cAMP

Palmitoleic acid plays a major role in the regulation of cancer / tumour cells in the human body. Palmitoleic acid is associated with reduced cytotoxity. It is observed in higher concentrations in breast cancer. This means it might have an important function in cancer and tumor development. It might interfere with certain chemotherapies which are cytotoxic to targeted cells. It might on the other hand protect the healthy body cells, other than those targeted from the cytotoxicity.

Palmitoleic acid has cyto-protective properties. It appears that palmitoleic acid protects cells against cytotoxicity, apoptosis, necrosis and the likes, thus preventing cell death and by expansion tissue degradation and tissue death. It can easily be seen how this is important in numerous cases, eg dermatological conditions where cells are damaged or dying (gangrene, MRSA, lesions, insect, snake and spider bites etc.) or in case of internal cell damage, such as oesophagal, gastric, intestinal, respiratory, as a result of reflux acid burns, poisoning, smoke damage (cigarette) chemical fumes (eg fire brigade associated), gastric ulcers, parasites and infections, and I BS.

Scientific research results support the importance of a balanced omega-7 saturation index Sl (palmitic:palmitoleic acid) against benign fibrocystic breast changes and the progression of proliferative changes to breast cancer. Further, it has been reported that the intake of omega-3 fatty acids has protective effects. Total omega-3 fatty acids, EPA, and the Sl for palmitic to palmitoleic acid were associated with significantly lower risk of breast cancer.

### FOOD / FEED

The influence of the diet on the metabolism and lipid peroxidation has been the subject of much research. It is clear that the relative balance between monounsaturated and long-chain polyunsaturated acyl chains in membrane bilayers is a fundamental determinant of metabolic rate of a species.

It is generally acknowledged that anti-oxidation is a very essential step to whole body health. By example, healthy and strong membranes are important for general health and well being. The composition according to the present invention may provide the fatty acid composition of the cell membrane with an additional amount of omega-4 and omega-7 fatty acids. A balanced shift in the ratios saturated fatty acids/mono-unsaturated fatty acids/poly-unsaturated fatty acids may be beneficial to make the cell membrane optimally resistant against peroxidase and cell ageing.

Palmitoleic acid protects the cells against cell death and increases insulin sensitivity. Further, the mediation of palmitoleic acid on blood cholesterol is important for the general health state of people. Thus, an omega fatty acid composition comprising one or more of a fatty acid selected from the group of omega-4 fatty acids and omega-7 fatty acids, **characterized in that** omega-7 and omega-4 fatty acids are present in at least 10% weight percentage of the total fatty acid fraction (TFAF) of the composition., either or not combined with other omega fatty acids may have very beneficial properties. Through its mediating/stabilizing part in cholesterol, there are additional benefits for the whole human's organism.

The use of algal omega-7 and omega-4 fatty acids can find many applications such as but not limited to preterm infant formulas, term infant formulas, nursing infant food, maternal food, geriatric food, parenteral nutrition, nutritional beverages and functional foods and beverages. Further, as multicellular organisms with multiple membranes, also animals can benefit from feed enriched with omega-7 and omega-4 fatty acids

As the benefits such as the anti-oxidative scavenging of omega-7 and omega-4 fatty acids and the subsequent beneficial effects through mediation of cholesterol have elaborately been described above, it is not surprising that the combination of the omega fatty acid composition according to the present invention combined with other bioactive compounds has a fortifying effect.

Advantageously, the composition also contains vitamins, preferably vitamins B3, B6 and C, and minerals, preferably zinc and magnesium.

Scientific studies support the hypothesis that the major antioxidant nutrients vitamin E and vitamin C, and beta-carotene (which may or may not be acting as an antioxidant in vivo), may play a beneficial role in prevention of several chronic disorders. These compounds are well known, and strategies for their protection in foods are already exploited by food technologies. The impact of other bioactive non-energy compounds, such as other carotenoids and flavonoids, such as astaxanthin, on human health may also have beneficial effects. Further, the composition may comprise other additive such as glucosamine, chondroitine, calcium, magnesium, selenium, hydrated silica, MSM, medium chain triglycerids, phospholipids, phytosterols, stanols, oryzanol, policosanol, resviratrols, retinol, selective serotonin reuptake inhibitors, astaxanthin, terpenes, terpenoids, isoprenoids, statins, sesamol, sesaminol, and polyphenols.

The term 'treating' as used herein includes within its scope reducing, delaying and/or preventing the above mentioned conditions.

According to a preferred embodiment of the present invention, omega-7 and/or omega-4 fatty acids can also be used as a carrier for therapeutic agents. As used herein, the term "therapeutic agent" refers to an agent that has activity in a biological system. Particularly useful classes of therapeutic agent include, but are not limited to, cough suppressants, such as dextromethorphan hydrobromide and codeine; antibiotics such as cephalosporin; antihistamines such as chlorpheniramine maleate, brompheniramine maleate, loratidine, astemizole, diclofenac sodium and terfenadine; decongestants such as pseudoephedrine and phenylephrine; antihypertensives such as ACE-inhibitors, verapamil, nifedipine, propanolol, metoprolol, metoprolol succinate, metoprolol fumarate, metoprolol, methylphenadate, tartarate; agents to treat attention deficit disorder/hyperactivity such as methylphenadate, d and/or I isomers of methylphenadate, amphetamines, d and/or I isomers of amphetamines, and combinations of amphetamines; calcium channel blockers such as verapamil, diltiazam, nifedipine, nimodipine, felodipine, nicardipine, isradipine and amlodipine; antidiabetic agents such as glipizide and ibromectin; proton pump inhibitors such as omeprazole; anti-convulsants and anti-epileptics such as valproate sodium, clonazepam, gabapetin, and topiramate; anti-depressives such as buspirone, fluoxeline, 5-hydroxytryptamine receptor agonist and antagonist; anti-migraines such as sumatreptan and dihydroergotamine; antipsychotics such as resperidone; antiemetics such as ondansetron; anti- heartburns such as cisapride; H2 receptor antagonists such as cimetidine, ranitidine, famotidine, nizatidine; carbamazepine; beta adrenergic receptor blockers; anti-Parkinson agents such as selegiline, carbidopa/levodopa, pergolide, bromocriptine, amantadine, trihexyphenidyl HCI; antiviral agents including antiherpesvirus agents such as acyclovir, famciclovir, valcyclovir, foscamet, ganciclovir; antiretroviral agents such as didanosine, stavudine, zalcitabine, zidovudine; and others such as amantadine, interferon alpha, ribavirin, rimantadine; anti Alzheimer's agents such as galantamine; and other therapeutic agents such as cimetidine, propiomazine, phenytoin, tacrine, propiazam, proplazam; vinca alkaloid.

The carrier(s), diluent(s) or excipient(s) must be "acceptable" in the sense of being compatible with the other ingredients of the formulation and not being deleterious to the recipient thereof. It will be appreciated that the amount of a compound of the invention required for use in treatment will vary not only with the particular compound selected but also with the route of administration, the nature of the condition for which treatment is required and the age and condition of the patient and will be ultimately at the discretion of the attendant physician or veterinarian. In general however a suitable dose will be in the range of from about 0.1 to about 60 mg/kg of body weight per day, alternatively in the range of 0.5 to 6 mg/kg/day, in a further alternative in the range of 1 to 20 mg/kg/day.

While it is possible that, for use in therapy, a compound or combination of the invention may be administered as the raw chemical, it is preferable to present the active ingredient as a pharmaceutical composition.

Pharmaceutical compositions include those suitable for oral, rectal, nasal, topical (including buccal and sub-lingual), transdermal, vaginal or parenteral (including intramuscular, sub-cutaneous and intravenous) administration or in a form suitable for administration by inhalation or insufflation. The formulations may, where appropriate, be conveniently presented in discrete dosage units and may be prepared by any of the methods well known in the art of pharmacy. All methods include the step of bringing into association the active compound with liquid carriers or finely divided solid carriers or both and then, if necessary, shaping the product into the desired formulation.

Pharmaceutical compositions suitable for oral administration may conveniently be presented as discrete units such as capsules, gels, slow releasers, patches, cachets, tablets and or carrier each containing a predetermined amount of the active ingredient; as a powder or granules; as a solution, a suspension or as an emulsion. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The compounds and combinations according to the invention may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions! or emulsions in oily or aqueous vehicles, and may contain formulatory agents such as suspending, stabilizing an/or dispersing agents. Alternatively, the active ingredient may be in powder form, obtained by aseptic isolation of sterile solid or by lyophilisation from solution, for constitution with a suitable vehicle, e.g. sterile, pyrogen-free water, before use.

The carrier will usually form from 5% to 99. 9%, preferably from 25% to 80% by weight of the fatty acid composition, and can, in the absence of other adjuncts, form the balance of the composition.

### TOPICAL PRODUCT EXAMPLES

All topical medicaments, prepared according to the invention, may be prepared in any conventional form or in other forms suitable for topical use on the skin or mucous membranes. These forms also include all forms with sustained release compositions or pre-dispersed plasters and other forms containing an acceptable carrier for the compounds.

The active components are generally incorporated in a dermatological acceptable carrier in conventional manner. The active components can suitably first be dissolved or dispersed in a portion of the water or another solvent or liquid to be incorporated in the composition. The preferred compositions are oil-in-water or water-in-oil emulsions.

The composition may be in the form of conventional skin-care products such as a cream, gel or lotion or the like. The composition can also be in the form of a so-called `washoff' product e. g. a bath or shower gel, possibly containing a delivery system for the actives to promote adherence to the skin during rinsing. Most preferably the product is a `leave-on' product; a product to be applied to the skin without a deliberate rinsing step soon after its application to the skin.

Cosmetic acceptable carriers and additives may include: forms of vaseline, oils, emollients, surfactants, humefactants, powders, water, preservatives, agents increasing the viscosity such as polysaccharides, polypeptides, fragrances, anti-foam agents, opacifiers, colorants, each in their amount to accomplish their typical function. The basic compositions of the various topical application forms described here are well within the reach of the skilled person.

The present invention will be further illustrated in the following examples, which are in no way intended to be limiting to the invention.

### Example 1

The formulation below describes an algal oil in water cream suitable for the topical product according to the present invention. The percentages indicated are by weight of the composition.

| | Wt% |
|---|---|
| Mineral oil | 4 |
| Palmitoleic acid | 2.3 |
| Hexadecadienoic acid | 1.15 |
| Brij 56* | 4 |
| Alfol 16RD** | 4 |
| Triethanolamine | 0.75 |
| Butane-1, 3-diol | 3 |
| Xanthan gum | 0.3 |
| Perfume | Qs |
| Butylated hydroxy toluene | 0.01 |
| Water | To 100 |

| | |
|---|---|
| *Brij 56 is cetyl alcohol POE ** Alfol 16RD is cetyl alcohol | |

### Example 2

The formulation below describes an emulsion cream according to the present invention.

| Full chemical name or CTFA name | Trade name | Wt% |
|---|---|---|
| Palmitoleic acid | | 2.7 |
| Hexadecadienoic acid | | 2.0 |
| Disodium EDTA | Sequesterene Na2 | 0.05 |
| Magnesium aluminium silicate | Veegum Ultra | 0.6 |
| Methyl paraben | Methyl paraben | 0.15 |
| Simethicone | DC Antifoam Emulsion | 0.01 |
| Butylene Glycol 1,3 | Butylene Glycol 1,3 | 3.0 |
| Hydroxyethylcellulose | Natrosol 250HHR | 0.5 |
| Glycerine, USP | Glycerine, USP | 2.0 |
| Xanthan gum | Keltrol 1000 | 0.2 |
| Triethanolamine | Triethanolamine (99%) | 1.2 |
| Stearic acid | Pristerene 4911 | 3.0 |
| Propyl paraben NF | Propyl paraben NF | 0.1 |
| Glyceryl hydrostearate | Naturechem GMHS | 1.5 |
| Stearyl alcolhol | Lanette 18 DEO | 1.5 |
| Isostearyl palmitate | Protachem ISP | 6.0 |
| C12-15 alcohols octanoate | Hetester FAO | 3.0 |
| Dimethicone | Silicone Fluid 200 (50cts) | 1.0 |
| Cholesterol NF | Cholesterol NF | 0.5 |
| Sorbitan stearate | Sorbitan stearate | 1.0 |
| Butylated hydroxytoluene | Embanox BHT | 0.05 |
| Tocopheryl acetate | Vitamin E acetate | 0.1 |
| PEG-100 stearate | Myrj 59 | 2.0 |
| Sodium stearoyl lactylate | Pationic SSL | 0.5 |
| Hydroxycaprylic acid | Hydroxycaprylic acid | 0.1 |
| Retinyl palmitate | Vitamin A Palmitate | 0.06 |
| Alpha-bisabolol | Alpha-bisabolol | 0.2 |
| Water, DI | | q.s. to 100 |

Both the above topical compositions of examples 1 and 2 provide a suitable cosmetic treatment which may improve the appearance of wrinkled, aged, photo-damaged, and/or irritated skin, when applied to skin that has deteriorated through the aging or photoaging or when applied to youthful skin to help prevent or delay such deteriorative changes.

### ORAL PRODUCTS EXAMPLES

### Example 3

The formulation below describes a nutritional preparation including algae omega-7 and omega-4 fatty acids extracts. A composition comprising the following constituents suitable for oral application is formed by: admixing palmitoleic acid, hexodecadienoic acid, and optionally policosanol comprising octacosanol to form a mixture; and processing the mixture to form tablets or capsules; wherein the composition includes:
490 mg palmitoleic acid;
70 mg hexadecadienoic acid
10 mg policosanol, whereof 6 mg octacosanol

### Example 4

The formulation below describes a nutritional preparation including 5-methyltetrahydrofolate, palmitoleic acid, Hexadecadienoic acid and optionally, a stool softener. A composition comprising the following constituents suitable for oral application is formed by: admixing 5-methyltetrahydrofolate, palmitoleic acid, hexadecadienoic acid, and optionally, docusate sodium to form a mixture; and processing the mixture to form tablets or capsules; wherein the composition includes:
600 µg 5-methyltetrahydrofolate;
250 mg palmitoleic acid;
100 mg hexadecadienoic acid; and
50 mg docusate sodium (optional)

Optionally, folic acid, calcium, iron and/or vitamin D3 can be added to the composition. The composition may be used for administration to preconceptional and pregnant women to reduce the risk of development defects during pregnancy and to enhance the development of an embryo or fetus. It may also be used to improve the nutritional status of the woman throughout pregnancy and in the postnatal period for both lactating and non-lactating mothers. In addition, the preparation may be used for administration to breast feeding mothers to provide newborns with essential nutrients and vitamins to aid in continued growth and maturity of the brain, nervous system, and retina and to assist in cognitive development. Furthermore, it may be used to prepare preconceptional supplement products for administration to preconceptional women to improve the nutritional status of the woman prior to conception.

The present invention also encompasses the use of the fatty acid composition and/or derivatives thereof in the preparation of a topical and/or oral composition for providing at least one of the above exemplified health care benefits.

The present invention is also directed to the use of the composition in at least 10% weight percentage of the total weight, preferably at least 30% and more preferably at least 50% weight percentage of the total weight.

A person skilled in the art will understand that the examples described above are merely illustrative in accordance with the present invention and not limiting the intended scope of the invention. Other applications of the present invention may also be considered.

## Claims

1. An omega fatty acid composition comprising one or more of a fatty acid selected from:
i) omega-4 fatty acid; and
ii) omega-7 fatty acid;
**characterized in that** i) and ii) are present in at least 10% weight percentage of the total fatty acid fraction (TFAF) of the composition.

2. The composition according to claim 1, whereby the omega fatty acids are extracted from algae.

3. The composition according to claim 2, whereby the omega fatty acids are extracted from diatoms.

4. A composition according to claim 1 for use in oral and/or topical products.

5. Use of a composition according to claim 4 in nutrients.

6. Use of a composition according to claim 4 for making a product for treating the cardiovascular, cerebral, nervous, gastro-intestinal, musculoskeletal, metabolic, respiratory and/or uro-genital system.

7. Use of a composition according to claim 4 for making a product for treating allergy disorders, inflammation or immunologic disorders, cancer and/or tumors.

8. Use of a composition according to claim 4 in dermatologic and/or cosmetic products.

9. A composition according to claim 1 to be used as an additive in food and/or feed products.

10. Use of a composition according to claim 1 in at least 10% weight percentage of the total weight.

11. The composition according to claim 1, further comprising at least one omega-3, omega-6, omega-9, omega-11 and/or omega-13 fatty acid.

12. A composition according to claim 10, whereby the ratio of omega-3 to omega-4 and omega-7 fatty acids ranges from 1:5 to 5:1.

13. A composition according to claim 1, further comprising glucosamine, chondroitine, MSM, antioxidants, carotenoids, vitamins, medium chain triglycerids, phospholipids, phytosterols, resviratrols, retinol, statins, selective serotonin reuptake inhibitors, stanols, oryzanol, policosanol, sesamol, sesaminol, flavonoids, polyphenols, astaxanthin, terpenes, terpenoids, isoprenoids, liquid silica, biotin, coenzyme QIO and/or hyaluronic acid.

14. Use of a composition according to claim 1 in capsules, gels, slow releasers, powders, emulsions, patches and/or carriers.

15. A process for obtaining a composition according to claim 2, **characterized in that** the omega fatty acids are cultivated and stored in an algae biomass, either or not followed by extraction.
